# EUROPEAN PATENT APPLICATION

(11) **EP 1 407 719 A2**
(43) Date of publication of application: **14.04.2004**
(21) Application number: 03256050.0
(22) Date of filing: 25.09.2003
(51) Int. Cl.: A61B 18/14

(54) **A surgical instrument**

(30) Priority: 08.10.2002 GB 0223348
(71) Applicant: GYRUS MEDICAL LIMITED, Cardiff CF3 0LT (GB)
(72) Inventor: Goble, Colin Charles Owen, Egham Surrey, TW20 OXE (GB)
(74) Representative: Blatchford, William Michael

(57) **Abstract**

An electrosurgical instrument for use in cutting and/or coagulating tissue includes a dielectric material (30), the dielectric material being positioned in the current pathway between the tissue-treatment regions of first and second electrodes (15,16). The dielectric coating acts to couple the RF signal into the tissue primarily by capacitive coupling, providing a more even heating of the tissue and the elimination of "hot spots". Examples of electrosurgical instruments employing such coated electrodes include forceps, scissors or scalpel blade instruments.

## Description

This invention relates to a bipolar electrosurgical instrument such as a forceps, scissors or scalpel blade. Such instruments are commonly used for the cutting and/or coagulation of tissue in surgical intervention, most commonly in "keyhole" or minimally invasive surgery, but also in "open" surgery.

Electrosurgical devices generally fall into two categories, monopolar and bipolar. In a monopolar device a radio frequency signal is supplied to an active electrode which is used to treat tissue at the target site, an electrical circuit being completed by a grounding pad which is generally a large area pad attached to the patient at a location remote from the target site. In contrast, in a bipolar arrangement both an active and a return electrode are present on the instrument, and the current flows from the active electrode to the return electrode, often by way of an arc formed therebetween. The present invention relates to bipolar devices.

For many electrosurgical devices the control of the maximum current density able to be delivered by the electrodes is of great importance. Devices such as forceps often have insulating stops to prevent shorting contact between the electrode faces. US-A-4492231 and US-A-5891142 together with WO-A-02/07627 are examples of these kinds of measure. The present invention seeks to provide an improvement over this type of electrosurgical device.

Accordingly there is provided a bipolar radio frequency electrosurgical instrument comprising at least first and second electrodes, each of the first and second electrodes having a tissue-treatment region wherein, in use, current flows in a pathway from the tissue-treatment region of one electrode to the tissue treatment region of the other electrode, and at least one dielectric element made of a dielectric material, the dielectric element having a tissue-contacting portion and being positioned in the current pathway between the tissue-treatment regions of the first and second electrodes, the dielectric element having a reactive impedance of less than 3,000 ohms/sq.mm. at 450kHz.

Dielectric materials have been used to partially coat electrodes such as patient plate return electrodes and cardiac stimulation paddles, as for example in US-A-5836942. The dielectric does not form the main pathway for current flow (merely masking the sharp edges of the electrode), and the dielectric properties of the material in US-A-5836942 are well outside the range of reactive impedances of the material referred to above. In contrast, in the present invention the RF signal supplied to the tissue is primarily transmitted by capacitive coupling. Therefore, in the event of a low resistance pathway being present between the electrodes, for example by a short circuit being set up by conductive tissue, conductive fluid or by the electrodes coming into contact one with another, the maximum current flow will be limited by the capacitive nature of the dielectric element. In effect, the dielectric element, which is associated with at least one of the electrodes, acts as a current density limiting element. Thus, even in the event of a short circuit between the electrodes at one point therebetween, the device will still be capable of functioning satisfactorily at other positions between the electrodes.

The dielectric element conveniently has a reactive impedance of between 700 and 2,500 ohms/sq. mm. and preferably between 800 and 2,340 ohms/sq.mm. at 450kHz. Conveniently, the dielectric material comprises a ceramic material, such as a barium titanate ceramic material. The bipolar radio frequency instrument is conveniently a pair of forceps, scissors, or a bipolar scalpel blade.

In one convenient arrangement, the tissue-treatment region of at least one of the electrodes is at least partially coated with the dielectric material. In some embodiments of the invention, notably forceps embodiments, the tissue-treatment regions of both of the first and second electrodes are at lease partially covered with the dielectric material. In such embodiments, the tissue-treatment region of at least one and preferably both of the electrodes is completely covered with the dielectric material.

The invention further resides in an electrosurgical instrument comprising a bipolar cutting blade, and a handpiece to which the blade is secured, the cutting blade comprises first and second electrodes, and an electrical insulator spacing apart the electrodes, each of the first and second electrodes having a tissue-treatment region, where, in use, current flows in a pathway from the tissue-treatment region of one electrode to the tissue treatment region of the other electrode, and at least one dielectric element made of a dielectric material, the dielectric element having a tissue-contacting portion and being positioned in the current pathway between the tissue-treatment regions of the first and second electrodes, the dielectric element having a reactive impedance of less than 3,000 ohms/sq.mm. at 450kHz.

In some embodiments the dielectric element is provided as a partial coating on one of the electrodes. In other embodiments the dielectric element is provided as a partial coating on the electrical insulator separating the electrodes.

The invention further resides in an electrosurgical system for treating tissue, the system comprising a bipolar radio frequency instrument comprising at least first and second electrodes, each of the first and second electrodes having a tissue-treatment region, and an electrosurgical generator adapted to supply a radio frequency output to the electrodes of the instrument at a frequency f, such that the current flows in a pathway from the tissue-treatment region of one of the electrodes to the tissue treatment region of the other electrode, and at least one dielectric element made of a dielectric material, the dielectric element having a tissue-contacting portion and being positioned in the current pathway between the tissue-treatment regions of the first and second electrodes, the dielectric element having a reactive impedance at the frequency f of less than 3,000 ohms/sq.mm. Thus, at the frequency supplied to the instrument by the generator, the dielectric element has a reactive impedance of less than 3,000 ohms/sq.mm. The frequency f is conveniently be one of the internationally recognised Industrial Scientific of Medical bands (ISM), which are currently 6.79MHz, 13.56MHz, 27.12MHz and 40.68MHz.

The invention also includes a bipolar radio frequency electrosurgical instrument comprising mutually adjacent first and second electrodes each having a tissue contact surface, wherein at least one of the electrodes comprises a dielectric layer applied to an electrically conductive base member, the dielectric layer forming the tissue contact surface and having a reactive impedance of less than 3000 ohms/sq.mm. at 450kHz. In the preferred embodiment, both the first and the second electrode comprise a conductive base member and a dielectric layer forming the tissue contact surface. Such an arrangement is particularly suited to electrosurgical forceps having a pair of jaws each of which comprises an electrode.

According to another aspect of the invention, an electrosurgical system for treating tissue comprises a bipolar radio frequency instrument and an electrosurgical generator adapted to supply radio frequency power to the instrument at an operating frequency f when the generator is connected to the instrument, wherein the instrument comprises first and second electrodes each having a tissue contact surface, at least one of the electrodes including an electrically conductive base member and a dielectric covering applied to the base member to form the tissue contact surface of the electrode, and wherein the dielectric layer has a reactive impedance of less than 3000 ohms per square millimetre of tissue contact surface area when receiving radio frequency power from the generator at the operating frequency.

The invention will now be described by way of example one, with reference to the accompanying drawings, in which;
Figure 1 is a schematic diagram of an electrosurgical system including an electrosurgical instrument in accordance with the present invention,
Figure 2 is a schematic cross-sectional view of an electrosurgical forceps in accordance with the present invention,
Figure 3 is a schematic close-up of the jaw region of the electrosurgical forceps of Figure 2,
Figure 4 is a schematic diagram shown an instrument which is a pair of bipolar scissors,
Figures 5 and 6 are schematic diagrams of an electrosurgical cutting blade,
Figure 7 is a schematic view of the cutting blade of Figures 5 and 6 modified in accordance with a first embodiment of the present invention, and
Figure 8 is a schematic view of the cutting blade of Figures 5 and 6 modified in accordance with a second embodiment of the present invention.

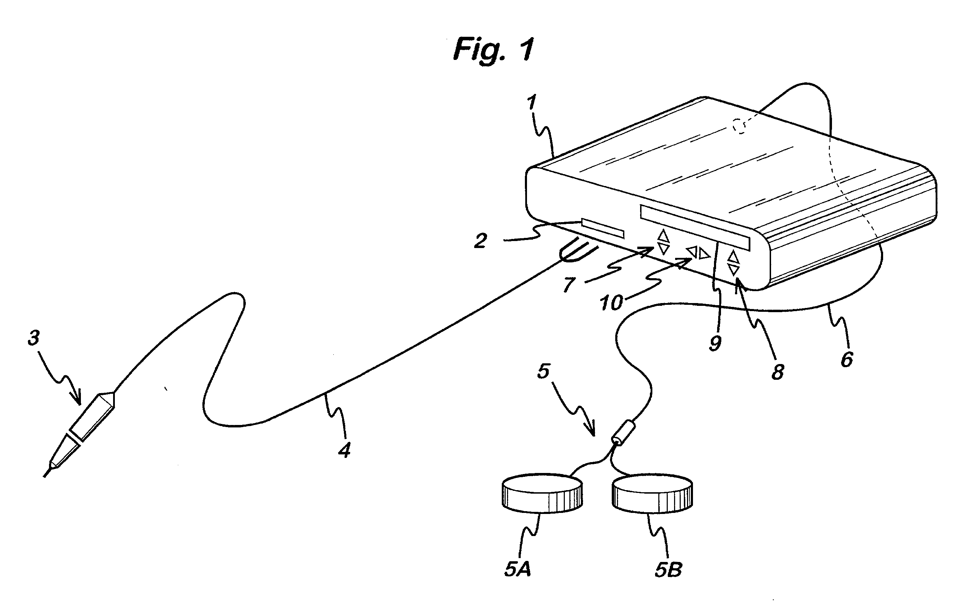

Referring to Figure 1, a generator 1 has an output socket 2 providing a radio frequency (RF) output for an instrument 3 via a connection cord 4. Activation of the generator may be performed from the instrument 3 via a connection in cord 4 or by means of a footswitch unit 5, as shown, connected to the rear of the generator by a footswitch connection cord 6. In the illustrated embodiment footswitch unit 5 has two footswitches 5A and 5B for selecting a coagulation mode and a cutting mode of the generator respectively. The generator front panel has push buttons 7 and 8 for respectively setting coagulation and cutting power levels, which are indicated in a display 9. Push buttons 10 are provided as an alternative means for selection between coagulation and cutting modes.

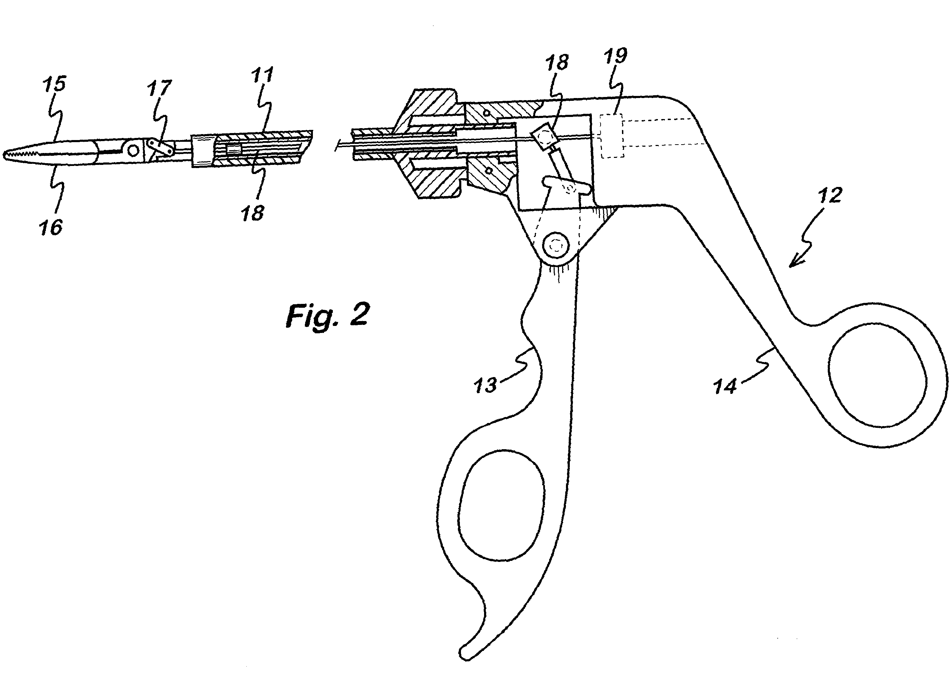

Referring to Figure 2, there is shown a bipolar coagulating forceps device, which is one device constituting the instrument 3 in Figure 1. The forceps comprises a tubular barrel 11 attached at its proximal end to a handle assembly 12, the handle assembly including first and second scissor handles 13 and 14, the handle 13 being pivotable with respect to the handle 14. At the distal end of the tubular barrel 11 is a pair of jaws 15 and 16, the jaws being pivotally movable one with respect to the other by means of a distal link assembly 17, operated by means a cable 18 running through the tubular barrel and attached to the handle 13 by means of a proximal link assembly 18. In this way, the pivotal movement of the handle 13 with respect to the handle 14 causes the jaws 15 and 16 to open and close with respect to one another. This type of forceps device is entirely conventional, and a more detailed description of such a device is contained in US-A-5342381 by way of example.

Jaws 15 and 16 are formed of steel and are coated with a 1 mm coating of a barium titanate ceramic dielectric material. The coating material is known commercially as Z5U, and is available as an industry standard dielectric material. The Z5U material has a dielectric constant of 11,000.

In use, tissue to be coagulated is held firmly between the jaws 15 and 16, and a coagulating radio frequency voltage is supplied to the jaws from the generator 1, via connector 19 at the rear of the instrument. The radio frequency signal is coupled into the tissue held between the jaws, heating it and causing the tissue to become coagulated. The dielectric coating on the jaws 15 and 16 controls the maximum current density in the region of the tissue, and ensures even heating of the tissue avoiding the generation of individual "hot spots" as can be produced by purely resistively coupled heating. This helps to guarantee that the tissue to be treated is coagulated rather than desiccated. Desiccation of tissue is undesirable, as the absence of electrolyte presents a high impedance to the RF generator, thereby preventing further RF energy from being supplied to the tissue. If tissue such as a blood vessel becomes desiccated around its outer region, it is possible that the further application of RF energy may fail to treat the inner region of the vessel, no matter how prolonged the treatment. The use of the dielectric material provides a more even heating action, maintaining the treatment temperature at a coagulation rather than a desiccation temperature, thereby avoiding this potential problem.

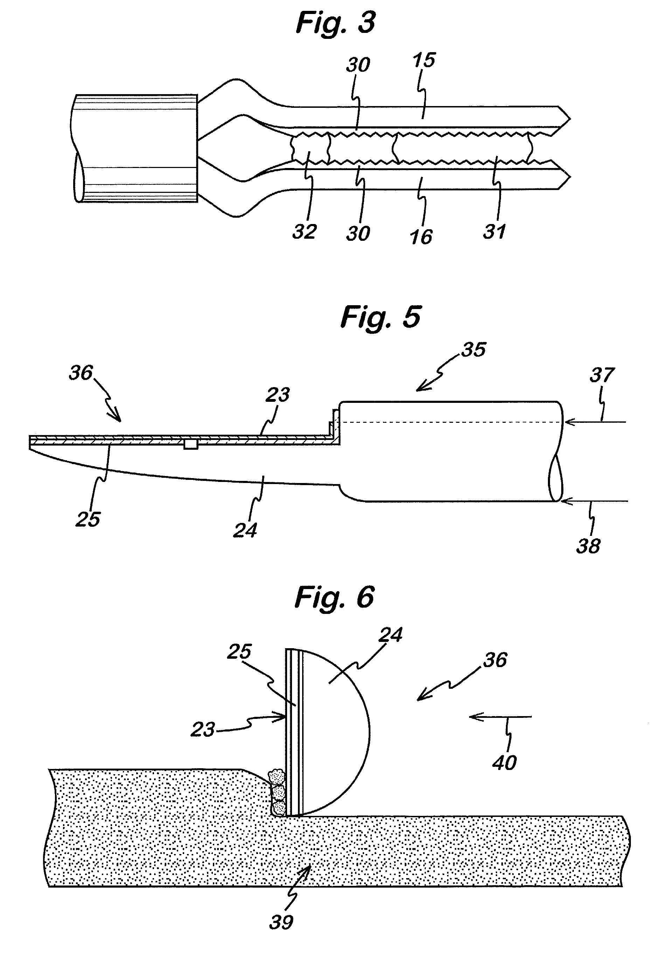

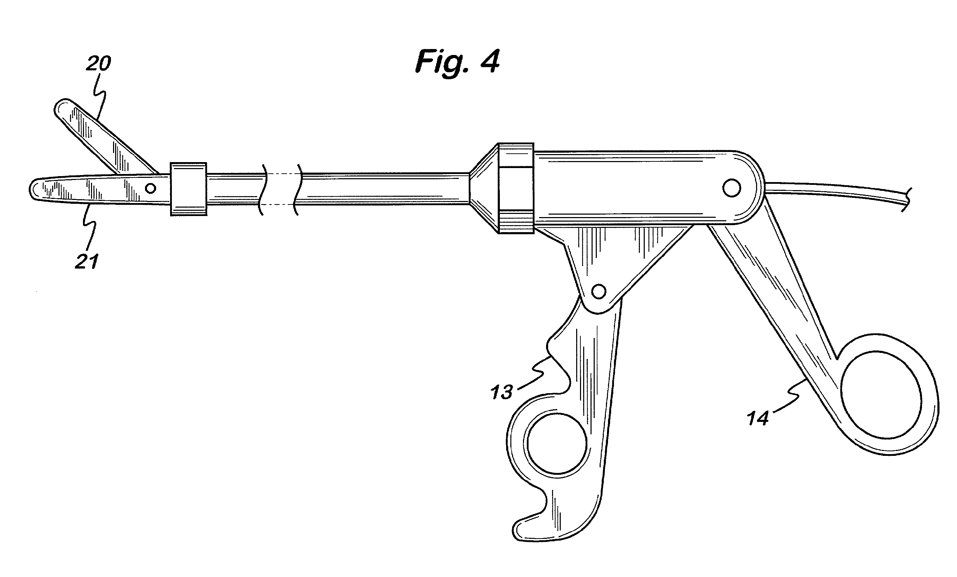

The dielectric nature of the material provides a further advantage, as will be explained with reference to Figure 3. Figure 3 shows jaws 15 and 16 with a coating 30 of a dielectric material such as Z5U applied thereto. A tissue vessel 31 is gripped between the jaws, but there is also a conductive fluid shown generally at 32. The conductive fluid can be saline, blood, or a mixture of the two, and serves to produce an unwanted low impedance electrical pathway between the jaws, akin to a short circuit. In other devices this can cause a problem, with all of the current being focused through the fluid 32 rather than through the tissue 31. However, with the dielectric nature of the coating 30, the RF energy is coupled capacitively rather than resistively from the jaws 15 and 16, and RF energy will still be coupled into the tissue 31 despite the presence of the fluid 32.

A further advantage of the dielectric coating 30 is that the energy coupled into the tissue will be automatically adjusted depending on the amount of tissue grasped between the jaws 15 and 16. As the dielectric coating limits the maximum current density in the region of the tissue, the rate at which RF energy is supplied to the tissue will depend on how much tissue is present. If a relatively large piece of tissue is grasped between the jaws 15 and 16, a relatively high power RF signal can be supplied to the tissue before the maximum current density is reached. However, if a relatively small piece of tissue is grasped between the jaws 15 and 16, the maximum current density will be reached more quickly, and further RF energy will not be coupled to the tissue.

Figure 4 shows an alternative device in which the jaws are in the form of cutting blades 20 and 21. In this bipolar scissors device, which is again entirely conventional apart from the dielectric material coating applied to the blades, the coating again provides improved control of current density helping to prevent the adherence of tissue to the blades. Such bipolar scissors devices can be used to both cut and coagulate tissue, and it is a common problem for their effectiveness to become impaired by the build-up of tissue on the blades thereof. The use of the dielectric material coating reduces this problem, and extends the operating life of the scissors device.

Figure 5 shows a further device which is in the form of a bipolar scalpel blade, as depicted in our co-pending WO-A-03/055402. The instrument 35 comprises a blade shown generally at 36 and including a generally flat first electrode 23, a larger second electrode 24, and an electrically insulating spacer 25 separating the first and second electrodes. The first electrode 23 is formed of stainless steel having a thermal conductivity of 18W/m.K (although alternative materials such as Nichrome alloy may also be used). The second electrode 24 is formed from a highly thermally-conducting material such as copper having a thermal conductivity of 400W/m.K (alternative materials including silver or aluminium). The surface of the second electrode 24 is plated with a biocompatible material such as a chromium alloy, or with an alternative non-oxidising material such as nickel, gold, platinum, palladium, stainless steel or tungsten disulphide. The spacer 25 is formed from a ceramic material such as aluminium oxide which has a thermal conductivity of 30W/m.K. Other possible materials for the spacer 25 are available which have a substantially lower thermal conductivity. These include boron nitride, PTFE, reinforced mica, silicon rubber or foamed ceramic materials.

A conductive lead 37 is connected to the first electrode 23, and a lead 38 is connected to the second electrode 24. The RF output from the generator 1 is connected to the blade 36 via the leads 37 and 38 so that a radio frequency signal having a substantially constant peak voltage (typically around 400V) appears between the first and second electrodes 23 and 24. Referring to Figure 6, when the blade 36 is brought into contact with tissue 39 at a target site, the RF voltage causes arcing between one of the electrodes and the tissue surface. Because the first electrode 23 is smaller in cross-sectional area, and has a lower thermal capacity and conductivity than that of the second electrode 24, the first electrode assumes the role of the active electrode and arcing occurs from this electrode to the tissue 39. Electrical current flows through the tissue 39 to the second electrode 24, which assumes the role of the return electrode. Cutting of the tissue occurs at the active electrode, and the blade may be moved through the tissue. The blade 36 may be used to make an incision in the tissue 39, or moved laterally in the direction of the arrow 40 in Figure 6 to remove a layer of tissue.

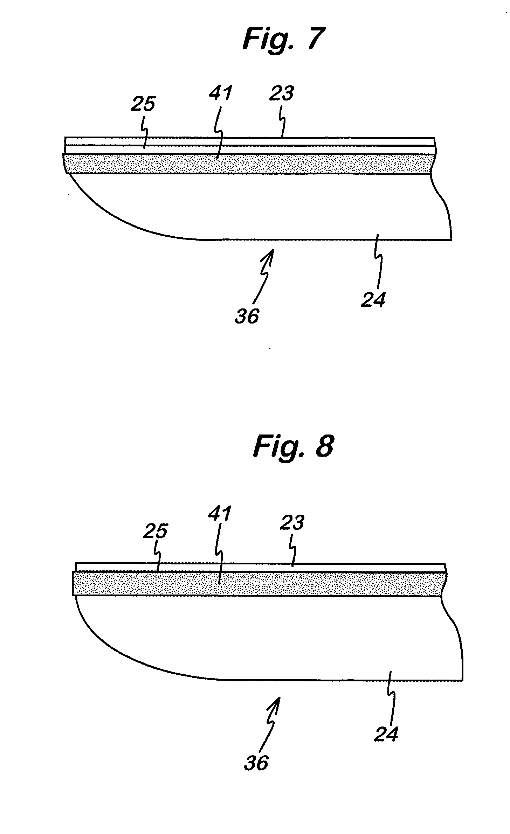

Figure 7 is an enlarged view of an end portion of the blade 36 showing how it is modified in accordance with the invention. In this drawing, the blade is viewed from the underside, i.e. looking onto the longitudinal cutting edge of the blade in a direction parallel to the major face of the first electrode 23 and perpendicular to the cutting edge, as in Figure 5. The first and second electrodes 23 and 24 are shown as before, together with the insulating spacer 25, which is shown as being somewhat thinner than in Figure 5. This is because a strip of the second electrode 24 is coated with a coating 41 of dielectric material having a higher dielectric constant than that of the spacer (generally at least 10 times that of the spacer). A preferred material for the coating 41 is Z5U. Each of the electrodes 23, 24 has a respective tissue treatment region forming part of the cutting edge. That of the first electrode 23, in this case the active electrode, is exposed, whereas that of the second electrode 24, the return electrode, is covered by the coating 41. The coating 41 extends as a band along the entire length of the blade underside, i.e. the cutting edge, and is applied to the second electrode 24 in the region which is adjacent the insulator 25. The coating 41 also extends over the second electrode 24 on the end face of the blade 36. It lies adjacent to and abutting the insulating spacer 25 along the underside of the blade and around its end. It follows that the coating 41 masks conductive surfaces of the second electrode 24 which would otherwise contact the tissue being treated, acting as a series reactive impedance in the RF current path between the second electrode 24 and the tissue.

An advantage conferred by the dielectric coating 41 is that it can allow the blade to be made smaller or flatter than previously shown in Figure 5. Vaporised tissue products tend to condense or become otherwise deposited on the electrodes, and tissue cut by the device can also become attached thereto. If the build-up of deposited material produces one or more conductive tracks across the insulating spacer 25, a short circuit can be produced between the electrodes 23 and 24 causing a concentration of current flow. One of the limitations on the design of the previous scalpel blade was the requirement to try to avoid this condition, and so the insulating spacer 25 was made broad enough to discourage or inhibit the formation of such conductive tracks. The use of a high dielectric constant material for the coating 41 on the second electrode 24 limits the maximum current density flowing between the electrodes 34 and 24, and means that the blade will continue to function even if a conductive track is formed. Thus the insulating spacer 25 can be made thinner, allowing for a flatter or smaller blade design.

Figure 8 shows an alternative embodiment in which the edge surface of the insulating spacer 25 is provided with the coating 41 of dielectric material rather than that of the second electrode 24. The coating 41 extends along the length of the spacer 25 and covers the end face thereof. The spacer 25 is thicker than in the embodiment of Figure 7, but as the current flowing from the first electrode 23 is coupled to the second electrode 24 via the dielectric coating 41, the dielectric covered spacer 25 has the effect of an extension of the second electrode 24, acting as an RF shunt impedance between the electrodes in parallel to the current path through tissue fluids adjacent the electrodes. The cutting action of the blade 36 is similar to that of Figure 7, even though the insulating spacer is wider.

Whichever embodiment is considered, the effect of the dielectric material is to place a maximum on the current density which can be generated between the bipolar electrodes. This serves to ensure that the device functions correctly, even if there are one or more low impedance pathways set up between the electrodes, such as by conductive material becoming attached to the device, or by the presence of fluid between the electrodes.

## Claims

1. A bipolar radio frequency electrosurgical instrument comprising at least first and second electrodes (15,16; 20,21; 23,24), each of the first and second electrodes having a tissue-treatment region wherein, in use, current flows in a pathway from the tissue-treatment region of one electrode to the tissue-treatment region of the other electrode, and at least one dielectric element (30;41) made of a dielectric material, the dielectric element having a tissue-contacting portion and being positioned in the current pathway between the tissue-treatment regions of the first and second electrodes, the dielectric element having a reactive impedance of less than 3,000 ohms/sq.mm. at 450kHz.

2. An instrument according to claim 1, **characterised in that** the dielectric element (30;41) has a reactive impedance of between 700 and 2,500 ohms/sq.mm. at 450kHz.

3. An instrument according to claim 2, **characterised in that** the dielectric element (30;41) has a reactive impedance of between 800 and 2,340 ohms/sq.mm. at 450kHz.

4. An instrument according to any preceding claim, **characterised in that** the dielectric element (30;41) is made of a ceramic material.

5. An instrument according to claim 4, **characterised in that** the ceramic material is a barium titanate material.

6. An instrument according to any preceding claim, **characterised in that** the dielectric element (30;41) comprises a dielectric coating at least partially covering the tissue-treatment region of one of the electrodes.

7. An instrument according to any of claims 1 to 5, **characterised by** first and second dielectric elements (30) comprising dielectric coatings at least partially covering the tissue-treatment regions of the first and second elements.

8. A bipolar radio frequency electrosurgical instrument according to any of claims 1 to 5, **characterised in that** the tissue-treatment region of at least one of the electrodes is completely covered with the dielectric material (30;41).

9. A bipolar radio frequency electrosurgical instrument according to any of claims 1 to 5, **characterised in that** the tissue-treatment region of both of the electrodes (15,16; 20,21) is completely covered with the dielectric material.

10. A bipolar radio frequency electrosurgical instrument according to claim 1, **characterised in that** the instrument is in the form of pair of forceps.

11. A bipolar radio frequency electrosurgical instrument according to claim 1, **characterised in that** the instrument is in the form of a scalpel blade.

12. An electrosurgical instrument comprising a bipolar cutting blade and a handpiece to which the blade is secured, the cutting blade comprising first and second electrodes (23,24), and an electrical insulator (25) spacing apart the electrodes, each of the first and second electrodes having a tissue-treatment region, wherein, in use, current flows in a pathway from the tissue-treatment region of one electrode to the tissue treatment region of the other electrode, and at least one dielectric element (41) made of a dielectric material, the dielectric element having a tissue-contacting portion and being positioned in the current pathway between the tissue-treatment regions of the first and second electrodes, the dielectric element having a reactive impedance of less than 3,000 ohms/sq. mm. at 450kHz.

13. An electrosurgical instrument according to claim 12, **characterised in that** the electrical insulator (25) is at least partially coated with the dielectric material.

14. An electrosurgical system for treating tissue, the system comprising a bipolar radio frequency instrument (3) comprising at least first and second electrodes (15,16; 20,21; 23,24), each of the first and second electrodes having a tissue-treatment region, and an electrosurgical generator (1) adapted to supply a radio frequency output to the electrodes of the instrument at a frequency f, such that current flows in a pathway from the tissue-treatment region of one of the electrodes to the other, and a dielectric element (30;41), the dielectric element having a tissue-contacting portion and being positioned in the current pathway between the tissue-treatment regions of the first and second electrodes, the dielectric element having a reactive impedance at the frequency f of less than 3,000 ohms/sq. mm.

15. An electrosurgical system for treating tissue, the system comprising a bipolar radio frequency instrument (3) comprising at least first and second electrodes (15,16; 20,21; 23,24), each of the first and second electrodes having a tissue-treatment region, and an electrosurgical generator (1) adapted to supply a radio frequency output to the electrodes of the instrument at a frequency of 6.79MHz, such that current flows in a pathway from the tissue-treatment region of one of the electrodes to the tissue treatment region of the other electrode, and at least one dielectric element (30;41) made of a dielectric material, the dielectric element having a tissue-contacting portion and being positioned in the current pathway between the tissue-treatment regions of the first and second electrodes, the dielectric element having a reactive impedance at the 6.79MHz of less than 3,000 ohms/sq. mm.

16. An electrosurgical system for treating tissue, the system comprising a bipolar radio frequency instrument (3) comprising at least first and second electrodes (15,16; 20,21; 23,24), each of the first and second electrodes having a tissue-treatment region, and an electrosurgical generator (1) adapted to supply a radio frequency output to the electrodes of the instrument at a frequency of 13.56MHz, such that current flows in a pathway from the tissue-treatment region of one of the electrodes to tissue treatment region of the other electrode, and at least one dielectric element (30;41) made of a dielectric material, the dielectric element having a tissue-contacting portion and being positioned in the current pathway between the tissue-treatment regions of the first and second electrodes, the dielectric element having a reactive impedance at the 13.56MHz frequency of less than 3,000 ohms/sq. mm.

17. An electrosurgical system for treating tissue, the system comprising a bipolar radio frequency instrument (3) comprising at least first and second electrodes (15,16; 20,21; 23,24), each of the first and second electrodes having a tissue-treatment region, and an electrosurgical generator (1) adapted to supply a radio frequency output to the electrodes of the instrument at a frequency of 27.12MHz, such that current flows in a pathway from the tissue-treatment region of one of the electrodes to the tissue treatment region of the other electrode, and at least one dielectric element (30;41) made of a dielectric material, the dielectric element having a tissue-contacting portion and being positioned in the current pathway between the tissue-treatment regions of the first and second electrodes, the dielectric element having a reactive impedance at the 27.12MHz frequency of less than 3,000 ohms/sq.mm.

18. An electrosurgical system for treating tissue, the system comprising a bipolar radio frequency instrument (3) comprising at least first and second electrodes (15,16; 20,21; 23,24), each of the first and second electrodes having a tissue-treatment region, and an electrosurgical generator (1) adapted to supply a radio frequency output to the electrodes of the instrument at a frequency of 40.68MHz, such that the current flows in a pathway from the tissue-treatment region of one of the electrodes to the tissue treatment region of the other electrode, and at least one dielectric element (30;41) made of a dielectric material, the dielectric element having a tissue-contacting portion and being positioned in the current pathway between the tissue treatment regions of the first and second electrodes, the dielectric element having a reactive impedance at the 40.68MHz frequency of less than 3,000 ohms/sq.mm.

19. An electrosurgical instrument comprising a bipolar tissue cutting blade and a handpiece to which the blade is secured, wherein the blade comprises a laminar combination of first and second electrically conductive electrodes (23,24) spaced apart by an intermediate insulating layer (25), the electrodes having neighbouring co-extensive edge portions forming tissue-treatment regions, and wherein the blade further comprises at least one dielectric element (41) formed as a tissue-contacting extension of the edge portion of the second electrode, the dielectric element being made of a dielectric material having a relative dielectric constant which is at least 10 times greater than that of the material of the intermediate layer.

20. An instrument according to claim 19, **characterised in that** the dielectric element (41) at least partially covers the edge portion of the second electrode.

21. An instrument according to claim 19, **characterised in that** the dielectric element (41) is a dielectric coating covering the tissue-treatment region of the second electrode.

22. An instrument according to claim 19, **characterised in that** the dielectric element (41) is an elongate element extending along the edge portion of the second electrode.

23. An instrument according to claim 19, **characterised in that** the insulating layer (25) has an edge portion co-extensive with the electrode edge portions and wherein the dielectric element (41) is an elongate element abutting and extending longitudinally along the edge portion of the second electrode, and at least partially covering the insulating layer edge portion.
